# EUROPÄISCHE PATENTSCHRIFT

(11) **EP 1 144 699 B1**
(45) Veröffentlichungstag und Bekanntmachung des Hinweises auf die Patenterteilung: **26.03.2003**
(21) Anmeldenummer: 99967938.4
(22) Anmeldetag: 14.12.1999
(51) Int. Cl.: C22B 3/28, C22B 34/30, C22B 23/00, C22B 34/36, C07C 211/11

(54) **VERFAHREN ZUR EXTRAKTION VON ANIONEN DER IVB BIS VIII METALLE MITTELS ALKYLSUBSTITUIERTER 1,3-DIAMINOPROPANE**
METHOD FOR EXTRACTING ANIONS OF IVB TO VIII METALS USING ALKYL-SUBSTITUTED 1,3-DIAMINOPROPANES
PROCEDE PERMETTANT D'EXTRAIRE DES ANIONS DES METAUX DES GROUPES IVB A VIII AU MOYEN DE 1,3-DIAMINOPROPANES ALKYL-SUBSTITUES

(30) Priorität: 23.12.1998 DE 19859683
(43) Veröffentlichungstag der Anmeldung: 17.10.2001
(73) Patentinhaber: H.C. Starck GmbH, 38642 Goslar (DE)
(72) Erfinder: GUTKNECHT, Wilfried, D-38642 Goslar (DE); MATHY, Wolfgang, D-38685 Langelsheim (DE)
(74) Vertreter: Zobel, Manfred, Dr.
(86) Internationale Anmeldenummer: EP9909914
(87) Internationale Veröffentlichungsnummer: WO00039350

(56) Entgegenhaltungen:
- EP-A- 0 199 905
- EP-A- 0 267 668
- EP-A- 0 417 870
- DE-A- 2 648 971
- US-A- 3 988 367
- US-A- 4 230 183

## Beschreibung

Die vorliegende Erfindung betrifft ein Verfahren zur Extraktion von in Form von Anionen, Metallaten oder anionischen Metallkomplexen vorliegenden Metallen der V. bis VIII. Gruppe des Periodensystems aus wässrigen Lösungen. Bei der Gewinnung reiner Metalle der V. bis VIII. Gruppe des Periodensystems ist die Extraktion aus wässrigen Lösungen mittels einer in der wässrigen Phase nicht löslichen organischen Phase, die ein Extraktionsmittel enthält, ein verbreitet eingesetzter Reinigungsschritt. Extraktionsverfahren werden einerseits zur Abtrennung von in Aufschlusslösungen vorhandenen aus den jeweiligen Rohstoffen resultierenden Verunreinigungen eingesetzt, andererseits aber auch zur Trennung von im Periodensystem benachbarten Elementen, die auf Grund ihrer chemischen Verwandschaft anderen Trennverfahren nicht oder nur schwer zugänglich sind. Von großem technischem Interesse sind insbesondere die Trennung der Paare W/Mo, Ta/Nb, Co/Ni sowie V/Cr. Darüber hinaus wird die Extraktion auch als Vehikel bei der chemischen Umsetzung beispielsweise von Natriumwolframat zu Ammoniumparawolframat eingesetzt, indem das Natriumwolframat in wässriger Lösung mit der organischen Phase kontaktiert wird, wobei Wolframationen in die organische Phase übergehen, und nach Trennung der organischen und wässrigen Phase die Wolframationen mit einer wässrigen Ammoniaklösung aus der organischen Phase gestrippt werden.

Bei der extraktiven Trennung wird die pH-Wert- und/oder Temperatur-abhängige selektive Beladungsfähigkeit und/oder die unterschiedliche Beladungskinetik der organischen Phase bezüglich verschiedener Ionen ausgenutzt.

Üblicherweise erfolgt die Extraktion in mehrstufigen "Mixer-Settler"-Kaskaden oder Kolonnen im Gegenstrom.

Neben der Forderung der Selektivität des Extraktionsmittel bzw. der organischen Phase in Bezug auf die zu trennenden Ionen sind an das Extraktionsmittel bzw. die organische Phase die Forderungen nach hoher Beladungsfähigkeit möglichst bei Raumtemperatur und bei angenähert neutralem pH-Wert zu stellen. Darüber hinaus soll das Extraktionsmittel eine möglichst geringe Wasserlöslichkeit und aus verfahrenstechnischen Gründen die organische Phase eine nicht zu hohe Viskosität aufweisen.

Die genannten Anforderungen werden von den bekannten Lösungsextraktionsverfahren nur unzureichend erfüllt, so dass der Wunsch nach verbesserten Extraktionsverfahren, insbesondere Extraktionsmitteln besteht. So erfolgt die Kobalt-Extraktion insbesondere mittels tertiärer Amine und quarternärer Ammoniumsalze, wobei eine Beladung von 10 bis 15 g/l Kobalt erzielt wird. Höhere Beladungen von 10 bis 25 g/l Kobalt werden durch Einsatz von Organophosphorsäuren (DEHPA Phosphonsäuren, Phosphinsäuren) erzielt, jedoch besteht hier das Risiko der Kontamination des Kobalt mit Phosphor. Ferner ist die Selektivität gegenüber Nickel gering. Die Molybdän-Extraktion wird überwiegend durch Einsatz sekundärer Amine als Extraktionsmittel bei Beladungen von 38 bis 42 g/l in der organischen Phase durchgeführt. Bei der Wolframextraktion mit sekundären Aminen werden Beladungen von 60 bis 70 g/l W erzielt, wogegen tertiäre und quarternäre Amine lediglich zu einer Beladung von 12 bis 15 g/l W führen.

Die DE-A 2 530 244 offenbart ein Extraktionsverfahren für Schwermetalle durch Komplexbildung mit Aminoalkanolen. Derartige Aminoalkanole weisen allerdings den Nachteil der für technische Anwendungen zu hohen Wasserlöslichkeit auf. Gemäß EP-A 505277 werden Eisen und Zirkon aus Lantaniden/Aktiniden mittels Propandiamiden abgetrennt. Die Veröffentlichung DU PREEZ, Mineral Processing and Extractive Metallurgy Review, 15 (1995) pp. 153 bis 161, offenbart den Einsatz von Tetra-substituierten Diaminen für Chlorokomplexe von ein- und zweiwertigen Metallen.

Es wurde nun gefunden, dass sich alkylsubstituierte 1,3-Diaminopropane in hervorragender Weise zur Extraktion von Anionen auf Basis der Metalle der V. bis VIII. Gruppe des Periodensystems aus deren wässrigen Lösungen eignen. Dabei wurden im Vergleich zu den bisher üblichen primären, sekundären oder tertiären Aminen und quarternären Ammoniumverbindungen eine überraschend hohe Beladungsfähigkeit gefunden.

Gegenstand der vorliegenden Erfindung ist demgemäß ein Verfahren zur Extraktion von Anionen auf Basis der Metalle der Gruppen V bis VIII des Periodensystems aus deren wässrigen Lösungen, das dadurch gekennzeichnet ist, dass als Extraktionsmittel Verbindungen der allgemeinen Formel 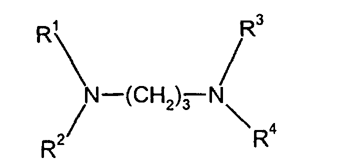 eingesetzt werden, wobei maximal zwei der R¹, R², R³ und R⁴ Wasserstoffatome und die übrigen gleiche oder verschiedene gegebenenfalls verzweigte Alkyl- oder Aminoalkylgruppen mit im Mittel mindestens 5 C-Atomen darstellen, wobei das Extraktionsmittel in Kombination mit einem organischen Lösungsmittel, das nicht wasserlöslich ist, eingesetzt wird.

Anionen auf Basis der Metalle der Gruppen V bis VIII des Periodensystems sind die Anionen der Metalle selbst, bevorzugt jedoch die entsprechenden Metallatanionen bzw. anionische Metallkomplexe.

Die Summe der C-Atome der R-Substituenten dividiert durch die Anzahl der Substituenten, die nicht Wasserstoffatome sind, soll mindestens 5 betragen, damit eine ausreichende Wasserunlöslichkeit gewährleistet wird. Bevorzugt soll die mittlere Anzahl der C-Atome der R-Substituenten, die nicht Wasserstoffatome sind, nicht mehr als 10 betragen, um die Viskosität des Extraktionsmittels im verfahrenstechnisch günstigen niedrigen Bereich zu halten. Bevorzugte erfindungsgemäße Extraktionsmittel gemäß der oben genannten Formel sind dadurch gekennzeichnet, dass R² und R⁴ Wasserstoffatome sind. Bevorzugte Substituenten R¹ und R³ sind gegebenenfalls verzweigte Nonylgruppen, insbesondere bevorzugt Isononyl-Gruppen.

Das Extraktionsmittel wird gemeinsam mit einem organischen Lösungsmittel, das nicht wasserlöslich ist, eingesetzt. Geeignet sind hochsiedende Kohlenwasserstoffgemische, die aliphatische, cycloaliphatische und aromatische organische Verbindungen aufweisen können. Insbesondere bevorzugt sind hochsiedende Testbenzine, wie sie beispielsweise von der Firma TOTAL unter der Bezeichnung Spirdane® HT erhältlich sind.

Bevorzugt wird das erfindungsgemäße Extraktionsmittel in Kombination mit Isodecanol (IDA) als Modifizierungsmittel eingesetzt.

Erfindungsgemäß bevorzugt werden organische Phasen eingesetzt, die 0,5 bis 95 Vol.-% Diaminopropan-Derivate, bis zu 99 Vol.-% Verdünnungsmittel und 0,5 bis 20 % Isodecanol enthalten. Besonders bevorzugt einsetzbare organische Phasen enthalten 10 bis 20 % des Diaminopropan-Derivates, 5 bis 15 % Isodecanol und 65 bis 85 % organisches Lösungsmittel.

Das erfindungsgemäße Extraktionsverfahren kann im Temperaturbereich von 15 bis 80°C durchgeführt werden, bevorzugt sind Temperaturen im Bereich von 20 bis 60°C.

Die wässrige Phase kann je nach den extrahierenden Ionen einen pH-Wert von 1 bis 10 aufweisen.

Das erfindungsgemäße Verfahren ist insbesondere zur Extraktion von Wolfram aus Molybdän und Wolfram enthaltenden Lösungen geeignet. Dabei können Wolframlaugen aus dem natronalkalischen Aufschluss nach Abtrennung der Verunreinigungen (insbesondere P, As, Si, Al, Ti, V, Nb, Ta, Sn) bei pH-Wert 8 bis 9 durch Fällung oder Ionenaustausch direkt zur Extraktion eingesetzt werden. Die extraktive Trennung von Wolfram und Molybdän erfolgt vorteilhaft bei einem pH-Wert von 7 bis 8,5 der wässrigen Phase, insbesondere bevorzugt bei einem pH-Wert von 7,3 bis 8,2. Zum Vergleich: Die Extraktion von Wolfram mit sekundären Aminen verlangt pH-Werte unterhalb von 6, zur Erzielung hoher Beladungen pH-Wert 2 bis 3; die Extraktion mit quarternären Amoniumverbindungen erfordert pH-Werte von 7 bis 7,5, jedoch lässt sich das Wolfram nur unvollständig mit Ammoniak strippen, so dass eine Molybdän/Wolfram-Trennung nur durch Sulfid-Zusatz möglich ist. Erfindungsgemäß werden bei pH-Werten um ca. 8 Beladungen der organischen Phase von ca. 120 g/l Wolfram und weniger als 2 mg/l Mo erreicht. Ein weiterer Vorteil des vergleichsweise hohen pH-Wertes des erfindungsgemäßen Verfahrens besteht darin, dass als Mineralsäure zur Einstellung und Einhaltung des pH-Wertes vorteilhaft Kohlendioxid eingesetzt werden kann. Zur Gewinnung von Ammoniumparawolframat (APW) wird die beladene organische Phase in an sich bekannter Weise nach Trennung von der wässrigen Phase mit Ammoniaklösung gestrippt.

Ein erfindungsgemäß weiter bevorzugtes Verfahren ist die extraktive Trennung von Kobalt und Nickel aus Kobalt- und Nickelionen enthaltenden wässrigen Lösungen. Vorzugsweise liegen die Kobalt- und Nickelionen in der wässrigen Lösung als Chloride vor. Die Extraktion von Kobalt aus der wässrigen Lösung erfolgt bei niedrigen pH-Wert, d. h. in Gegenwart freier Salzsäure in einer Konzentration von vorzugsweise 150 bis 250 g/l freier HCI, so dass das Kobalt als Chlorokomplex vorliegt.

### Beispiele

Die wässrige Feedlösung und die organische Phase (OP) wurden in einen Scheidetrichter eingefüllt, intensiv vermischt und durch Stehenlassen die Phasen getrennt.

Bei der (simulierten) zweistufigen Extraktion wurde die OP 2-mal mit frischer Feedlösung in Kontakt gebracht. Die Extraktionsversuche wurden bei Raumtemperatur (25°C) durchgeführt.

### Beispiel 1:

| | |
|---|---|
| OP | 20 % N,N-Bis-(isononyl)-1,3-diaminopropan |
| | 10 % Isodecanol |
| | 70 % Spirdane HT |
| Feedlösung | 90 g/l Co als Chlorid |
| | 10 g/l Ni als Chlorid |
| | 200 g/l freie HCl |
| Mengenverhältnis OP | Feed = 1:1 |
| Beladung OP nach 2-stufiger Extraktion | 30,8 g/l Co |
| | 0,15 g/l Ni. |

### Beispiel 2:

| | |
|---|---|
| OP | wie Beispiel 1 |
| Feedlösung | 91,7 g/l Mo als Na₂MoO₄ |
| Mengenverhältnis OP | Feed = 1:1 |
| pH | 7,3, eingestellt mit H₂SO₄ |
| OP-Beladung nach 2-stufiger Extraktion | 43 g/l Mo. |

### Beispiel 3

| | |
|---|---|
| OP | wie Beispiel 1 |
| Feedlösung | 111 g/l W als Na₂WO₄ |
| | 60 mg/l Mo |
| Mengenverhältnis OP | Feed = 1:1 |
| pH | 7,5, eingestellt mit H₂SO₄ |
| OP-Beladung nach 2-stufiger Extraktion | 122 g/l W |
| | < 2 mg/l Mo. |

### Beispiel 4:

| | |
|---|---|
| OP | wie Beispiel 1 |
| Feedlösung | 93,7 g/l V als Na₂VO₃ |
| Mengenverhältnis Feed | OP = 2:1 |
| pH | 6,3, eingestellt mit H₂SO₄ |
| OP-Beladung nach 1-stufiger Extraktion | 60 g/l V. |

### Beispiel 5

| | |
|---|---|
| OP | wie Beispiel 1 |
| Feedlösung | 35 g/l Cr als Na₂Cr₂O₇ |
| Mengenverhältnis Feed | OP = 1:1 |
| pH | 1,8 eingestellt mit H₂SO₄ |
| OP-Beladung nach 1-stufiger Extraktion | 33 g/l Cr. |

### Beispiel 6

| | |
|---|---|
| OP | wie Beispiel 1 |
| Feedlösung (HF-sauer) | 103 g/l Ta |
| | 62 g/l Nb |
| | 25 g/l Ti |
| Mengenverhältnis Feed | OP = 1:1 |
| OP-Beladung nach 1-stufiger Extraktion | 66,6 g/l Ta |
| | 33,1 g/l Nb |
| | 3,8 g/l Ti. |

### Beispiel 7:

| | |
|---|---|
| OP | 10 % N,N-Bis-(isononyl)-1,3-diaminopropan |
| | 10 % IDA |
| | 80 % Spirdane HT |
| Feed | 60 g/l W als Na₂WO₄ |
| | 90 mg/l Mo |
| Mengenverhältnis Feed | OP = 1:1 |

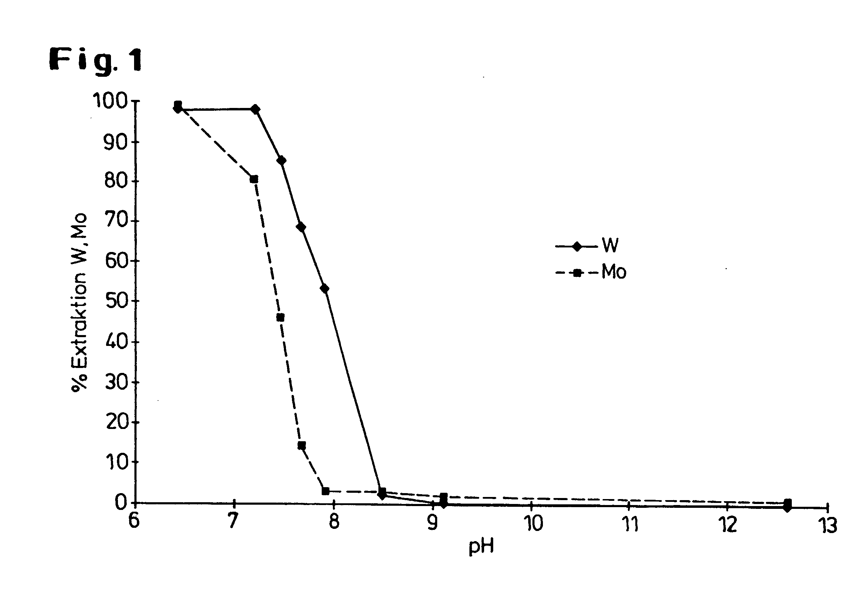

pH - Einstellung mit H₂SO₄ (variabel)
Beladung OP nach 1-stufiger Extraktion: Siehe Fig. 1.

Fig. 1 zeigt den %-Anteil der Metalle, die in die OP übergegangen sind, in Abhängigkeit vom pH-Wert.

## Patentansprüche

1. Verfahren zur Extraktion von Anionen auf Basis der Metalle der Gruppen V bis VIII des Periodensystems aus deren wässrigen Lösung, **dadurch gekennzeichnet, dass** als Extraktionsmittel Verbindungen der allgemeinen Formel 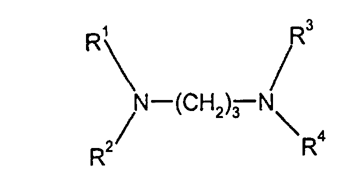 eingesetzt werden, wobei maximal zwei der R¹, R², R³ und R⁴ Wasserstoffatome und die übrigen gleiche oder verschiedene gegebenenfalls verzweigte Alkyl-oder Aminoalkylgruppen mit im Mittel mindestens 5 C-Atomen darstellen, wobei das Extraktionsmittel in Kombination mit einem organischen Lösungsmitel, das nicht wasserlöslich ist, eingesetzt wird.

2. Verfahren nach Anspruch 1, **dadurch gekennzeichnet, dass** R² und R⁴ Wasserstoffatome sind.

3. Verfahren nach Anspruch 1 oder 2, **dadurch gekennzeichnet, dass** R¹ und R³ gegebenenfalls verzweigte Nonylgruppen sind.

4. Verfahren nach Anspruch 1, **dadurch gekennzeichnet, dass** als Extraktionsmittel N,N-Bis-(isononyl)-1,3-diaminopropan eingesetzt wird.

5. Verfahren nach einem der Ansprüche 1 bis 4, **dadurch gekennzeichnet, dass** als organisches Lösungsmittel hochsiedende Kohlenwasserstoffgemische eingesetzt werden.

6. Verfahren nach einem der Ansprüche 1 bis 5, **dadurch gekennzeichnet, dass** das Extraktionsmittel in Kombination mit Isodecanol als Modifizierungsmittel eingesetzt wird.

7. Verfahren nach einem der Ansprüche 1 bis 6, **dadurch gekennzeichnet, dass** die wässrige Lösung Wolfram- und Molybdänionen enthält und Wolfram bei einem pH-Wert von 7,5 bis 8,5 extrahiert wird.

8. Verfahren nach einem der Ansprüche 1 bis 6, **dadurch gekennzeichnet, dass** die wässrige Lösung Kobalt und Nickel-haltige Ionen enthält und Kobalt extrahiert wird.

## Claims

1. Process for the extraction of anions based on metals of groups V to VIII of the Periodic Table from their aqueous solution, **characterized in that** extractants used are compounds of the general formula 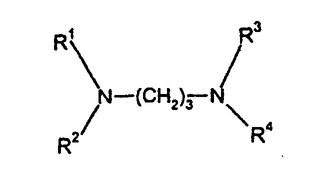 where not more than two of R¹, R², R³ and R⁴ are hydrogen atoms and the others are identical or different, branched or unbranched alkyl or amino alkyl groups having on average at least 5 carbon atoms, with the extractant being used in combination with an organic solvent which is not soluble in water.

2. Process according to Claim 1, **characterized in that** R² and R⁴ are hydrogen atoms.

3. Process according to Claim 1 or 2, **characterized in that** R¹ and R³ are branched or unbranched nonyl groups.

4. Process according to Claim 1, **characterized in that** the extractant used is N,N-bis(isononyl)-1,3-diaminopropane.

5. Process according to any of Claims 1 to 4, **characterized in that** the organic solvent used is a high-boiling hydrocarbon mixture.

6. Process according to any of Claims 1 to 5, **characterized in that** the extractant is used in combination with isodecanol as modifier.

7. Process according to any of Claims 1 to 6, **characterized in that** the aqueous solution contains tungsten and molybdenum ions and tungsten is extracted at a pH of from 7.5 to 8.5.

8. Process according to any of Claims 1 to 6, **characterized in that** the aqueous solution contains cobalt- and nickel-containing ions and cobalt is extracted.

## Revendications

1. Procédé d'extraction d'anions à base des métaux des groupes V à VIII du système périodique à partir de leur solution aqueuse, **caractérisé en ce qu'**on utilise comme agent d'extraction des composés de formule générale 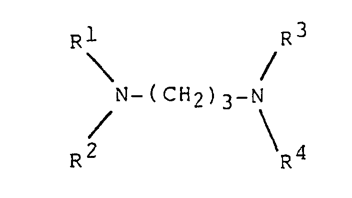 dans laquelle au maximum deux parmi R¹, R², R³ et R⁴ représentent des atomes d'hydrogène et les autres des groupements alkyle ou aminoalkyle identiques ou différents le cas échéant ramifiés avec en moyenne au moins 5 atomes de carbone, l'agent d'extraction étant utilisé en combinaison avec un solvant organique qui n'est pas soluble dans l'eau.

2. Procédé selon la revendication 1, **caractérisé en ce que** R² et R⁴ sont des atomes d'hydrogène.

3. Procédé selon la revendication 1 ou 2, **caractérisé en ce que** R¹ et R³ sont des groupements nonyle le cas échéant ramifiés.

4. Procédé selon la revendication 1, **caractérisé en ce qu'**on utilise comme agent d'extraction du N,N-bis-(isononyl)-1,3-diaminopropane.

5. Procédé selon l'une des revendications 1 à 4, **caractérisé en ce qu'**on utilise comme solvant organique des mélanges d'hydrocarbures à haut point d'ébullition.

6. Procédé selon l'une des revendications 1 à 5, **caractérisé en ce qu'**on utilise comme agent de modification l'agent d'extraction en combinaison avec de l'isodécanol.

7. Procédé selon l'une des revendications 1 à 6, **caractérisé en ce que** la solution aqueuse contient des ions tungstène et molybdène et l'on extrait du tungstène à un pH de 7,5 à 8,5.

8. Procédé selon l'une des revendications 1 à 6, **caractérisé en ce que** la solution aqueuse contient du cobalt et des ions contenant du nickel et l'on extrait du cobalt.
